# FASCICULE DE BREVET EUROPEEN

(11) **EP 3 102 321 B1**
(45) Date de publication et mention de la délivrance du brevet: **18.09.2019**
(21) Numéro de dépôt: 15706886.7
(22) Date de dépôt: 04.02.2015
(51) Int. Cl.: B01J 19/00, G01N 31/10

(54) **DISPOSITIF D'ÉVALUATION D'AU MOINS UN CRITÈRE DE PERFORMANCE DE CATALYSEURS HÉTÉROGÈNES**
VORRICHTUNG ZUR AUSWERTUNG VON MINDESTENS EINEM LEISTUNGSKRITERIUM VON HETEROGENEN KATALYSATOREN
DEVICE FOR EVALUATION OF AT LEAST ONE PERFORMANCE CRITERION OF HETEROGENEOUS CATALYSTS

(30) Priorité: 06.02.2014 FR 1450923
(43) Date de publication de la demande: 14.12.2016
(73) Titulaire: Centre National de la Recherche Scientifique, 75016 Paris (FR); Ecole Centrale De Lille, 59651 Villeneuve d'Ascq (FR); UNIVERSITE DES SCIENCES ET TECHNOLOGIES DE LILLE, 59655 Villeneuve d'Ascq Cedex (FR)
(72) Inventeur: DUMEIGNIL, Franck, F-59273 Fretin (FR); PAUL, Sébastien, F-59158 Thun St Amand (FR); DUHAMEL, Louise, F-59650 Villeneuve D'ascq (FR); FAYE, Jérémy, F-59110 La Madeleine (FR); MIQUEL, Pierre, B-1020 Bruxelles (BE); CAPRON, Mickaël, F-59830 Bachy (FR); DUBOIS, Jean-Luc, F-69390 Millery (FR)
(74) Mandataire: Lavoix
(86) Numéro de dépôt international: PCT/FR2015/050263
(87) Numéro de publication internationale: WO 2015/118263

(56) Documents cités:
- EP-A2- 1 108 467
- WO-A1-02/092220
- FR-A1- 2 795 513
- US-A1- 2002 127 146

## Description

La présente invention a pour objet un dispositif de criblage catalytique à haut débit, compact et économique, destiné à évaluer les performances de catalyseurs hétérogènes.

La catalyse hétérogène représente la branche de la catalyse dans laquelle le catalyseur se trouve sous une autre phase, au sens thermodynamique du terme, que les réactifs et les produits de la réaction catalysée. Le catalyseur est typiquement à l'état solide et catalyse une réaction dans laquelle les réactifs et les produits sont en phase liquide ou en phase gaz.

Il est utile d'évaluer les performances d'un catalyseur. Pour ce faire, il existe plusieurs critères de performance qu'on peut chiffrer. Par exemple, on peut déterminer le taux de conversion d'un réactif, c'est-à-dire le nombre de moles de réactifs ayant réagi divisé par le nombre de moles de ce réactif initialement présentes dans le réacteur (cas d'un réacteur fermé) ou le rapport du débit molaire de ce réactif ayant réagi sur le débit molaire de ce réactif entrant dans le réacteur (cas d'un réacteur ouvert), ou encore le rendement en produits de réaction.

WO 02/092220 décrit un ensemble réacteur permettant d'analyser le courant effluent provenant d'au moins un réacteur à flux continu, cet ensemble réacteur comprenant au moins un réacteur à flux continu, au moins un analyseur permettant de soumettre le courant d'effluent à une procédure d'analyse, chaque sortie du réacteur étant connectée à au moins un analyseur par un conduit d'effluent.

EP 1 108 467 décrit un réacteur particulièrement adapté à une utilisation dans l'évaluation combinatoire de catalyseurs. Plusieurs réacteurs peuvent être facilement assemblés en un réseau pour l'évaluation simultanée d'un certain nombre de catalyseurs.

FR 2 795 513 décrit un appareillage automatique de test multi-réacteurs de réaction chimique en présence éventuelle d'un catalyseur permettant l'évaluation rapide et simultanée de plusieurs jeux de conditions opératoires ainsi que l'acquisition de données sur l'avancement de la réaction et sur les performances de catalyseurs solides.

US 2002/127146 décrit une nouvelle vanne d'injection de gaz destinée à injecter des charges de gaz dans une phase mobile ou un courant porteur, et décrit également des réseaux de vannes d'injection parallèles capables d'injecter plusieurs échantillons de manière sensiblement simultanée et un procédé d'injection d'échantillons de gaz discrets à une pression contrôlée dans un chromatographe en phase gazeuse à haute résolution.

Des dispositifs d'évaluation de la performance de catalyseurs hétérogènes sont notamment connus du document WO 02/092219.

Ces dispositifs sont toutefois sophistiqués, encombrants et particulièrement onéreux.

La présente invention vise à remédier à ces inconvénients.

Elle propose en particulier un dispositif d'évaluation d'au moins un critère de performance de catalyseurs hétérogènes, comprenant :
- au moins une source de réactif,
- au moins une zone de réaction munie d'au moins un catalyseur et reliée à au moins une source de réactif, de manière à réaliser dans chaque zone de réaction une réaction de catalyse hétérogène entre chaque catalyseur présent dans la zone de réaction et le ou les réactifs issus de chaque source de réactif reliée à la zone de réaction, et
- des moyens d'évaluation d'au moins un critère de performance de catalyseurs hétérogènes.

Le dispositif selon l'invention comprend en outre un chromatographe en phase gazeuse et chaque zone de réaction est située dans un injecteur du chromatographe.

Ainsi, au lieu d'utiliser un réacteur de laboratoire habituellement employé pour les mesures de performances catalytiques, le dispositif selon l'invention repose sur le détournement de l'usage d'un injecteur d'un chromatographe en phase gazeuse pour l'utiliser en tant que réacteur catalytique à lit fixe. On peut ainsi utiliser simultanément plusieurs injecteurs en parallèle, ce qui permet d'accélérer les tests catalytiques en adoptant une méthodologie de criblage catalytique à haut débit. En outre, le dispositif présente l'avantage d'une réversibilité totale d'utilisation si l'utilisateur ne souhaite plus l'utiliser en tant que cribleur catalytique, mais à nouveau dans sa fonction d'origine de chromatographe.

Le dispositif comprend de préférence au moins deux zones de réaction.

Chaque catalyseur peut être à l'état solide et chaque réactif peut être à l'état gazeux.

Le critère de performance peut être choisi parmi le taux de conversion d'un réactif et le rendement en produits de réaction.

Chaque zone de réaction est avantageusement un insert de l'injecteur.

Chaque insert peut contenir un lit catalytique.

Le dispositif peut comprendre plusieurs injecteurs disposés en parallèle.

Le dispositif peut comprendre un module apte à régler indépendamment la température et la pression de chaque zone de réaction (i.e. chaque injecteur) et les débits d'alimentation de chaque zone de réaction.

Le chromatographe en phase gazeuse peut comprendre en outre un système d'échantillonnage, au moins une colonne chromatographique et au moins un système de détection, notamment un système de détection classiquement utilisé dans les chromatographes en phase gazeuse, par exemple un détecteur à ionisation de flamme (FID en langue anglaise), un catharomètre (TCD en langue anglaise) ou tout autre détecteur connu de l'homme de l'art.

Le dispositif peut comprendre une source de gaz vecteur, le gaz vecteur étant destiné à diluer et acheminer le ou les réactifs dans chaque zone de réaction.

Le ou les réactifs et le gaz vecteur entrant dans chaque zone de réaction peuvent être issus d'un évaporateur suivi d'un système de répartition des flux.

L'invention a également pour objet un procédé d'évaluation d'au moins un critère de performance de catalyseurs hétérogènes, mettant en oeuvre le dispositif décrit ci-dessus.

Le procédé selon l'invention comprend :
- dans chaque zone de réaction, une étape de réaction de catalyse hétérogène entre le(s) catalyseur(s) présent(s) dans la zone de réaction et le(s) réactif(s) issu(s) de chaque source de réactif reliée à la zone de réaction, et
- une étape d'évaluation d'au moins un critère de performance de chaque catalyseur, notamment dans chaque zone de réaction.

L'invention a également pour objet l'utilisation d'un dispositif décrit ci-dessus comme cribleur catalytique ou comme chromatographe, le dispositif pouvant être utilisé de manière réversible comme cribleur catalytique ou comme chromatographe

D'autres caractéristiques et avantages de la présente invention apparaîtront plus clairement à la lecture de la description suivante donnée à titre d'exemple illustratif et non limitatif et faite en référence aux dessins annexés sur lesquels :
- la figure 1 illustre schématiquement un dispositif d'évaluation de la performance d'un catalyseur hétérogène selon l'invention,
- la figure 2 est une vue en coupe longitudinale d'un injecteur utilisé dans le dispositif selon l'invention, et
- la figure 3 est une vue en coupe longitudinale d'un insert de l'injecteur.

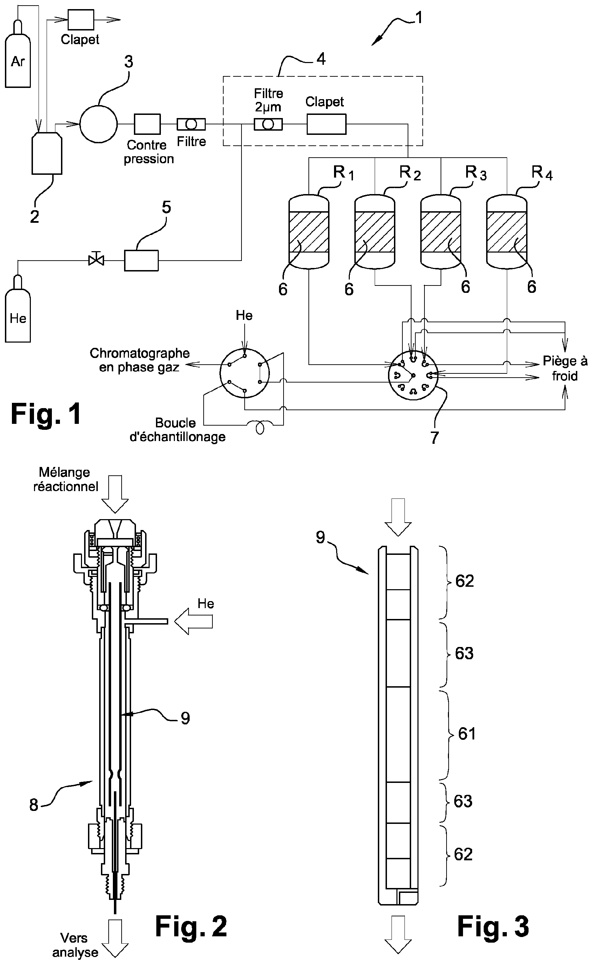

La figure 1 illustre un système 1 de criblage catalytique, appliqué à titre d'exemple à la conversion du méthanol. Un réservoir 2 d'une contenance d'un litre est rempli aux trois quarts de sa contenance avec du méthanol à 99,99%. Ce réservoir 2 est légèrement pressurisé sous argon à l'aide d'un cylindre Ar afin de dégazer le réactif (c'est-à-dire évacuer l'oxygène et l'azote dissous à température ambiante et pression atmosphérique) et également favoriser l'alimentation d'une pompe 3.

Le méthanol liquide est introduit dans un évaporateur 4 selon un débit prédéterminé (par exemple 100 µL.min⁻¹), l'évaporateur 4 étant simultanément alimenté en gaz vecteur (par exemple de l'hélium, à l'aide d'un cylindre He, à un débit de 160 mL.min⁻¹) au moyen d'un régulateur de débit massique 5. Les deux composés (l'hélium gazeux et le méthanol liquide) entrent dans l'évaporateur 4 qui comprend un tube rempli de carbure de silicium, dont le diamètre des particules est par exemple de 125 µm, et qui est maintenu à 120 °C. L'objectif est à cet endroit du dispositif 1 de vaporiser le méthanol et d'assurer l'homogénéité du mélange réactionnel gazeux à répartir sur les différents réacteurs. A titre d'exemple, quatre réacteurs R₁, R₂, R₃, R₄ ont été représentés sur la figure 1.

La sortie de l'évaporateur 4 est connectée à un répartiteur de flux muni d'une entrée et quatre sorties auxquelles sont reliés quatre tubes de silice fondue, de diamètre interne égal à 0.1 mm et d'une longueur de 40 cm, afin de générer une perte de charge individuelle trente fois supérieure à celle produite par le lit catalytique (2,25.10³ Pa par réacteur R₁, R₂, R₃, R₄). La perte de charge générée par ce répartiteur de flux est d'environ 9 à 10 bar.

Le mélange réactionnel est ainsi issu d'un module. Le module, qui vient s'imbriquer juste au-dessus des réacteurs R₁, R₂, R₃, R₄, permet de préparer le mélange d'alimentation qui va être injecté dans les réacteurs R₁, R₂, R₃, R₄ et de le répartir équitablement entre les différentes voies. On peut y préparer un mélange de réactif gazeux mais aussi vaporiser des liquides avant mélange avec des gaz et introduction dans les réacteurs. Le module est donc une sorte de four comprenant des évaporateurs, des tubes, des zones de mélange, des organes de contrôle et de régulation des débits, des éléments chauffants comme des résistances électriques. La configuration de ce module pourra être adaptée en fonction des réactions à étudier.

Le module permet notamment la régulation de la température de chaque réacteur R₁, R₂, R₃, R₄ et des débits d'alimentation. On peut aussi contrôler la pression de la réaction en ajoutant un déverseur en sortie de réacteur. Un logiciel peut assurer le contrôle du module, par exemple avec des entrées de consignes de température, de débits, ou encore l'indication de valeurs réelles. La régulation de la température peut être un contrôle extérieur ou un contrôle assuré par le chromatographe lui-même.

Conformément à l'invention, les réacteurs R₁, R₂, R₃, R₄ du dispositif 1 d'évaluation de la performance du catalyseur sont des injecteurs d'un chromatographe en phase gazeuse. Le reste du chromatographe peut être utilisé ou ne pas être utilisé.

Un chromatographe en phase gazeuse comprend typiquement :
- un four, qui permet une programmation de température ajustable et qui peut également être équipé d'un système de refroidissement rapide;
- un système d'injection, qui va permettre d'introduire et de rendre volatil l'échantillon à analyser. L'injection peut se faire d'une manière manuelle ou automatique à l'aide d'un échantillonneur;
- une colonne, sur laquelle les différentes molécules de l'échantillon injecté vont être séparées suivant leurs affinités avec la phase stationnaire de ladite colonne;
- un système de détection, qui va permettre de mesurer le signal émis par les différentes molécules, de pouvoir les identifier et de les quantifier après calibration;
- un système de détendeur-régulateur pour les gaz utilisés (hélium, hydrogène, azote et air comprimé). Sur les chromatographes modernes, on trouve des systèmes électroniques pour la régulation des gaz qui sont également purifiés par des cartouches filtrantes.

Le principe de fonctionnement du chromatographe est le suivant. L'échantillon (un liquide volatil ou un gaz) est d'abord introduit dans l'injecteur placé en tête de colonne par l'intermédiaire d'un échantillonneur ou d'une microseringue qui va traverser une pastille en caoutchouc, appelée septum, pour se retrouver dans une petite chambre en amont de la colonne appelée insert. L'injecteur est traversé par le gaz porteur et porté à une température appropriée à la volatilité de l'échantillon.

Ensuite, une fois rendus volatils, les différents composés de l'échantillon vont être emportés par le gaz porteur (ou gaz vecteur) à travers la colonne et se séparer les uns des autres en fonction de leur affinité avec la phase stationnaire. La phase stationnaire peut être un liquide peu ou non volatil (chromatographie gaz-liquide) ou un solide adsorbant (chromatographie gaz-solide). Dans les deux cas, la phase stationnaire va provoquer un phénomène de rétention chromatographique avec les différents composés appelés solutés. Plus le composé a d'affinité avec la phase stationnaire, plus il mettra de temps à sortir de la colonne. La grandeur expérimentale brute est appelée temps de rétention. C'est le temps qui s'écoule entre l'injection de l'échantillon et l'apparition du signal maximum du soluté au détecteur. Pour favoriser le transport de tous les composés à travers la colonne (élution), il faut déterminer la bonne température du four. En général, la température doit être légèrement supérieure à la température d'ébullition des composés, de manière à ce que les composés ne sortent pas trop tôt, ce qui aurait pour conséquence d'avoir leurs pics confondus avec celui du temps mort. On peut travailler en conditions isothermes, c'est-à-dire avec une température fixe durant toute l'analyse ou avec un programme de température qui varie.

À la sortie de la colonne, les composés rencontrent un élément essentiel qui est appelé détecteur. Cet élément évalue en continu la quantité de chacun des constituants séparés au sein du gaz porteur grâce à la mesure de différentes propriétés physiques du mélange gazeux. Le détecteur envoie un signal électronique vers un enregistreur qui dessinera les courbes de chaque pic en fonction de leur intensité (courbe de type Gaussienne). L'ensemble des pics est appelé chromatogramme.

Un chromatographe en phase gazeuse peut comprendre plusieurs voies d'analyse, notamment de deux à quatre, qui sont des ensembles injecteur+colonne+détecteur.

Les injecteurs sont logés dans un bloc métallique dont la température est régulée afin d'assurer une bonne homogénéité thermique du système. L'échantillon sera vaporisé et les solutés traverseront l'injecteur à travers un tube en verre (parfois métallique) appelé liner en langue anglaise (ou insert), grâce au gaz porteur, jusqu'à la tête de la colonne. L'intérêt de l'insert est de retenir les constituants non volatils de l'échantillon, impropres par nature à la chromatographie.

Dans le cas de l'utilisation de l'injecteur pour la détermination de performances catalytiques, chaque insert est rempli des catalyseurs solides à tester, sous forme de poudre, et est parcouru en continu par un mélange réactionnel gazeux.

Tel qu'illustré à la figure 1, le mélange réactionnel est ensuite dirigé simultanément vers les quatre réacteurs R₁, R₂, R₃, R₄, et est ainsi mis en contact avec un lit catalytique 6 à une température donnée. Le flux en sortie de chaque réacteur R₁, R₂, R₃, R₄ est, via une vanne multi-position 7, soit analysé par l'intermédiaire d'une boucle d'injection connectée à un système analytique externe au dispositif décrit ici, soit collecté à l'aide d'un piège à froid de type barboteur pour récupérer les produits désirés. L'évaluation de la performance du catalyseur peut être effectuée à l'aide d'un autre chromatographe en phase gazeuse ou à l'aide du même chromatographe en phase gazeuse que celui accueillant le système de criblage catalytique.

Conformément à l'invention, on détourne l'utilisation conventionnelle d'un chromatographe en phase gazeuse en utilisant l'insert 9 de l'injecteur 8 en tant que réacteur catalytique Rᵢ (figure 2).

Cette opération est réalisée en remplaçant le contenu de l'insert 9, habituellement de la laine minérale, par le lit catalytique 6 comprenant le catalyseur 61, ainsi que par exemple de la laine de quartz 62 et du carbure de silicium 63, tel qu'illustré à la figure 3. A titre d'exemple, l'insert 9 est donc totalement vidé pour être rempli par successivement :
- de la laine de quartz 62, pour assurer la stabilité du lit catalytique,
- du carbure de silicium 63 (125µm),
- le catalyseur 61 (200mg) mélangé avec du carbure de silicium (200mg),
- du carbure de silicium 63 (125µm),
- de la laine de quartz 62.

La présente invention est décrite plus en détail par l'exemple suivant, auquel elle n'est cependant pas limitée.

### Exemple : évaluation des performances catalytiques d'une alumine pour la déshydratation du méthanol

La déshydratation du méthanol en diméthyléther s'effectue selon la réaction suivante :

CH₃OH → CH₃OCH₃ + H₂O

### Conditions expérimentales

Le catalyseur testé est une alumine gamma γ-Al₂O₃ commercialisée par la société Alfa Aesar.

Le gaz vecteur est un mélange contenant 1% molaire de Kr dans l'He, dans lequel est évaporé le méthanol afin d'obtenir les proportions molaires suivantes pour le mélange réactionnel : MeOH/He/Kr: 27.4/71.9/0.7 (%mol).

La somme des débits gazeux est de 33000 mL.h⁻¹.g⁻¹ à 25°C et sous pression atmosphérique.

### Stabilité de la composition du mélange réactionnel

Avant chaque réaction, une série de six analyses par réacteur est effectuée pour vérifier la stabilité de la composition du mélange réactionnel. Le nombre de moles de méthanol indiqué pour chaque réacteur dans le tableau 1 est une moyenne des six analyses :

**Tableau 1**

| | R₁ | R₂ | R₃ | R₄ |
|---|---|---|---|---|
| n_{MeOH} (10⁻⁹ mol) | 668.9 | 677.7 | 666.9 | 667.9 |
| écart type (10⁻⁹ mol) | 16.4 | 20.2 | 16.4 | 15.9 |
| écart type relatif (%) | 2.5 | 3.0 | 2.5 | 2.4 |

On observe une bonne stabilité pour l'ensemble des réacteurs.

### Conversion du méthanol

Le taux de conversion du méthanol, ainsi que l'écart type relatif entre les réacteurs pour six analyses par réacteur, est indiqué dans le tableau 2 :

**Tableau 2**

| | R₁ | R₂ | R₃ | R₄ | moyenne | écart type relatif (%) |
|---|---|---|---|---|---|---|
| Conversion (%) | 83.4 | 84.0 | 82.6 | 83.0 | 83.3 | 0.7 |

On constate que la reproductibilité du taux de conversion du méthanol d'un réacteur à l'autre est excellente.

### Rendement en diméthyléther

Le rendement en diméthyléther pour chaque réacteur est indiqué dans le tableau 3 :

**Tableau 3**

| | R₁ | R₂ | R₃ | R₄ | moyenne | écart type relatif (%) |
|---|---|---|---|---|---|---|
| Rendement en diméthyléther (%) | 87.0 | 83.4 | 85.5 | 85.7 | 85.4 | 1.7 |

On observe une bonne reproductibilité des performances catalytiques entre les différents réacteurs.

L'invention permet ainsi d'obtenir un dispositif économique, compact et simple de mesure des performances des catalyseurs hétérogènes, notamment en phase gazeuse. Les différents injecteurs du chromatographe, détournés de leur application d'origine pour devenir des réacteurs, sont utilisables simultanément en parallèle ce qui permet de fortement accélérer les tests catalytiques en utilisant la méthodologie du criblage catalytique à haut débit. La technologie utilisée présente en outre l'avantage d'une réversibilité totale d'utilisation si on ne souhaite plus l'utiliser comme cribleur catalytique mais à nouveau dans sa fonction originelle de chromatographe. On peut en outre tester simultanément plusieurs catalyseurs, ou un seul catalyseur dans des conditions opératoires différentes (température ou temps de contact par exemple). On réduit ainsi significativement le temps nécessaire pour effectuer les mesures des performances catalytiques des catalyseurs et on diminue donc le temps de mise au point d'un nouveau catalyseur hétérogène.

## Revendications

1. Dispositif (1) d'évaluation d'au moins un critère de performance de catalyseurs hétérogènes, **caractérisé en ce qu'**il comprend :
- au moins une source de réactif (2), et
- un chromatographe en phase gazeuse doté d'au moins un injecteur (8), et **en ce que** ledit au moins un injecteur (8) englobe au moins un insert formant zone de réaction (9) munie d'au moins un catalyseur (61) et relié à ladite au moins une source de réactif (2), de manière à réaliser dans chaque zone de réaction (9) une réaction de catalyse hétérogène entre chaque catalyseur (61) présent dans la zone de réaction (9) et le ou les réactifs issus de chaque source de réactif (2), ledit dispositif comprenant en outre des moyens d'évaluation d'au moins un critère de performance de catalyseurs hétérogènes.

2. Dispositif (1) selon la revendication 1, **caractérisé en ce qu'**il comprend au moins deux zones de réaction (9).

3. Dispositif (1) selon la revendication 1 ou 2, **caractérisé en ce que** chaque catalyseur (61) est à l'état solide et **en ce que** chaque réactif est à l'état gazeux.

4. Dispositif (1) selon l'une des revendications 1 à 3, **caractérisé en ce que** le critère de performance est choisi parmi le taux de conversion d'un réactif et le rendement en produits de réaction.

5. Dispositif (1) selon l'une des revendications 1 à 4, **caractérisé en ce que** chaque insert (9) contient un lit catalytique (6).

6. Dispositif (1) selon l'une des revendications 1 à 5, **caractérisé en ce qu'**il comprend plusieurs injecteurs (8) disposés en parallèle.

7. Dispositif (1) selon l'une des revendications 1 à 6, **caractérisé en ce qu'**il comprend un module apte à régler indépendamment la température et la pression de chaque zone de réaction (9) et les débits d'alimentation de chaque zone de réaction (9).

8. Dispositif (1) selon l'une des revendications 1 à 7, **caractérisé en ce qu'**il comprend un système d'échantillonnage, au moins une colonne chromatographique et au moins un système de détection.

9. Dispositif (1) selon l'une des revendications 1 à 8, **caractérisé en ce qu'**il comprend une source de gaz vecteur, le gaz vecteur étant destiné à diluer et acheminer le ou les réactifs dans chaque zone de réaction (9).

10. Dispositif (1) selon la revendication 9, **caractérisé en ce que** le ou les réactifs et le gaz vecteur entrant dans chaque zone de réaction (9) sont issus d'un évaporateur (4) suivi d'un système de répartition des flux.

11. Procédé d'évaluation d'au moins un critère de performance de catalyseurs hétérogènes, mettant en oeuvre un dispositif (1) selon la revendication 1, **caractérisé en ce qu'**il comprend :
- dans chaque zone de réaction (9), une étape de réaction de catalyse hétérogène entre le(s) catalyseur(s) (61) présent(s) dans la zone de réaction (9) et le(s) réactif(s) issu(s) de chaque source de réactif (2) reliée à la zone de réaction (9), et
- une étape d'évaluation d'au moins un critère de performance de chaque catalyseur (61).

12. Utilisation d'un dispositif (1) selon l'une des revendications 1 à 11 comme cribleur catalytique ou comme chromatographe.

13. Utilisation d'un dispositif (1) selon l'une des revendications 1 à 11, le dispositif (1) pouvant être utilisé de manière réversible comme cribleur catalytique ou comme chromatographe.

## Patentansprüche

1. Vorrichtung (1) zur Auswertung von wenigstens einem Performancekriterium von heterogenen Katalysatoren, **dadurch gekennzeichnet, dass** sie aufweist:
- wenigstens eine Reagenz-Quelle (2) und
- einen Gaschromatographen, welcher mit mindestens einem Injektor (8) versehen ist, und wobei besagter mindestens ein Injektor (8) mindestens einen Einsatz aufweist, welcher eine Reaktionszone (9) bildet, die ausgestattet ist mit mindestens einem Katalysator (61), und verbunden ist mit besagter wenigstens einer Reagenz-Quelle (2), in der Art, dass eine heterogene, katalytische Reaktion in jeder Reaktionszone (9) zwischen jedem Katalysator (61), der präsent ist in der Reaktionszone (9), und dem oder den Reagenzien, welche aus jeder Reagenz-Quelle (2) stammen, realisiert wird, wobei besagte Vorrichtung weiterhin Auswertungsmittel von wenigstens einem Performancekriterium von heterogenen Katalysatoren umfasst.

2. Vorrichtung (1) gemäß Anspruch 1, **dadurch gekennzeichnet, dass** sie wenigstens zwei Reaktionszonen (9) aufweist.

3. Vorrichtung (1) gemäß Anspruch 1 oder 2, **dadurch gekennzeichnet, dass** jeder Katalysator (61) von festem Zustand ist und dass jedes Reagenz von gasförmigem Zustand ist.

4. Vorrichtung (1) gemäß einem der Ansprüche 1 bis 3, **dadurch gekennzeichnet, dass** das Performancekriterium ausgewählt ist aus der Konversionskurve eines Reagenzes und der Produktausbeute der Reaktion.

5. Vorrichtung (1) gemäß einem der Ansprüche 1 bis 4, **dadurch gekennzeichnet, dass** jeder Einsatz (9) ein katalytisches Bett (6) enthält.

6. Vorrichtung (1) gemäß einem der Ansprüche 1 bis 5, **dadurch gekennzeichnet, dass** sie mehrere Injektoren (8) aufweist, welche parallel angeordnet sind.

7. Vorrichtung (1) gemäß einem der Ansprüche 1 bis 6, **dadurch gekennzeichnet, dass** sie ein Modul aufweist, welches fähig ist, unabhängig die Temperatur und den Druck von jeder Reaktionszone (9) und die Zufuhrmengen von jeder Reaktionszone (9) zu regeln.

8. Vorrichtung (1) gemäß einem der Ansprüche 1 bis 7, **dadurch gekennzeichnet, dass** sie ein System der Probenentnahme, wenigstens eine Chromatographiesäule und wenigstens ein System zur Detektion aufweist.

9. Vorrichtung (1) gemäß einem der Ansprüche 1 bis 8, **dadurch gekennzeichnet, dass** sie eine Trägergasquelle aufweist, wobei das Trägergas dazu bestimmt ist, das oder die Reagenzien in jeder Reaktionszone (9) zu verdünnen und zu befördern.

10. Vorrichtung (1) gemäß Anspruch 9, **dadurch gekennzeichnet, dass** das oder die Reagenzien und das Trägergas, welche in jede Reaktionszone (9) eintreten, von einem Evaporator (4) stammen, gefolgt von einem System der Strömungsverteilung.

11. Verfahren zur Auswertung von wenigstens einem Performancekriterium von heterogenen Katalysatoren, welches eine Vorrichtung (1) gemäß Anspruch 1 anwendet, **dadurch gekennzeichnet, dass** es aufweist:
- in jeder Reaktionszone (9), einen Schritt der heterogenen, katalytischen Reaktion zwischen dem/den Katalysator(en) (61), welche(r) präsent ist/sind in der Reaktionszone (9), und dem/den Reagenz/Reagenzien, welche(s) von jeder Reagenz-Quelle (2) stammt/stammen, welche verbunden ist mit der Reaktionszone (9), und
- einen Schritt der Auswertung von wenigstens einem Performancekriterium von jedem Katalysator (61).

12. Verwendung einer Vorrichtung (1) gemäß einem der Ansprüche 1 bis 11 als katalytisches Sieb oder als Chromatograph.

13. Verwendung einer Vorrichtung (1) gemäß einem der Ansprüche 1 bis 11, wobei die Vorrichtung (1) auf reversible Art als katalytisches Sieb oder als Chromatograph benutzt werden kann.

## Claims

1. Device (1) for evaluating at least one performance criterion of heterogeneous catalysts, **characterised in that** it comprises:
- at least one reagent source (2), and
- a gas chromatograph equipped with at least one injector (8), and **in that** said at least one injector (8) includes at least one insert forming a reaction zone (9) which is provided with at least one catalyst (61) and is connected to said at least one reagent source (2), so as to carry out in each reaction zone (9) a heterogeneous catalysis reaction between each catalyst (61) present in the reaction zone (9) and the reagent(s) coming from each reagent source (2), said device further comprising means for evaluating at least one performance criterion of heterogeneous catalysts.

2. Device (1) according to claim 1, **characterised in that** it comprises at least two reaction zones (9).

3. Device (1) according to claim 1 or 2, **characterised in that** each catalyst (61) is in the solid state, and **in that** each reagent is in the gaseous state.

4. Device (1) according to any one of claims 1 to 3, **characterised in that** the performance criterion is chosen from the rate of conversion of a reagent and the yield of reaction products.

5. Device (1) according to any one of claims 1 to 4, **characterised in that** each insert (9) contains a catalytic bed (6).

6. Device (1) according to any one of claims 1 to 5, **characterised in that** it comprises a plurality of injectors (8) arranged in parallel.

7. Device (1) according to any one of claims 1 to 6, **characterised in that** it comprises a module capable of adjusting independently the temperature and the pressure of each reaction zone (9) and the feed rates of each reaction zone (9).

8. Device (1) according to any one of claims 1 to 7, **characterised in that** it comprises a sampling system, at least one chromatography column and at least one detection system.

9. Device (1) according to any one of claims 1 to 8, **characterised in that** it comprises a carrier gas source, the carrier gas being intended to dilute and transport the reagent(s) in each reaction zone (9).

10. Device (1) according to claim 9, **characterised in that** the reagent(s) and the carrier gas entering each reaction zone (9) come from an evaporator (4) followed by a flow distribution system.

11. Method for evaluating at least one performance criterion of heterogeneous catalysts, using a device (1) according to claim 1, **characterised in that** it comprises:
- in each reaction zone (9), a step of heterogeneous catalysis reaction between the catalyst(s) (61) present in the reaction zone (9) and the reagent(s) coming from each reagent source (2) connected to the reaction zone (9), and
- a step of evaluating at least one performance criterion of each catalyst (61).

12. Use of a device (1) according to any one of claims 1 to 11 as a catalyst screener or as a chromatograph.

13. Use of a device (1) according to any one of claims 1 to 11, the device (1) being capable of being used in a reversible manner as a catalyst screener or as a chromatograph.
